(19) Europäisches Patentamt
European Patent Office
Office européen des brevets

(11) Veröffentlichungsnummer: **0 012 108**
**B1**

# (12) EUROPÄISCHE PATENTSCHRIFT

(45) Veröffentlichungstag der Patentschrift: **28.07.82**

(51) Int. Cl.³: **C 07 C 43/295,** C 07 C 43/29

(21) Anmeldenummer: **79810154.9**

(22) Anmeldetag: **15.11.79**

(54) **Verfahren zur Herstellung von 4,5-Dichlor-2-(4-chlorphenoxy)-phenol und 4,5-Dichlor-2-(4-chlorphenoxy)-anisol.**

(30) Priorität: **21.11.78 CH 11919/78**

(43) Veröffentlichungstag der Anmeldung:
**11.06.80 Patentblatt 80/12**

(45) Bekanntmachung des Hinweises auf die Patenterteilung:
**28.07.82 Patentblatt 82/30**

(84) Benannte Vertragsstaaten:
**BE CH DE FR GB**

(56) Entgegenhaltungen:

**AT-B-245 555**
**CH-A-428 759**
**DE-A-1 793 664**
**DE-A-2 005 883**
**DE-A-2 849 856**
**US-A-3 904 696**

(73) Patentinhaber: **CIBA-GEIGY AG, Patentabteilung Postfach, CH-4002 Basel (CH)**

(72) Erfinder: **Weis, Claus D., Dr., Bünenmattweg 3, CH-4148 Pfeffingen (CH)**

EP 0 012 108 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von 4,5-Dichlor-2-(4-chlorphenoxy)-phenol und 4,5-Dichlor-2-(4-chlorphenoxy)-anisol

Die vorliegende Erfindung betrifft ein Verfahren zur Herstellung von 4,5-Dichlor-2-(4-chlorphenoxy)-phenol sowie ein Verfahren zur Herstellung des als Zwischenprodukt anfallenden 4,5-Dichlor-2-(4-chlorphenoxy)-anisols.

4,5-Dichlor-2-(4-chlorphenoxy)-phenol (= 4,4',5-Trichlor-2-hydroxydiphenyläther) ist, ebenso wie eine Reihe weiterer halogenierter 2-Hydroxydiphenyläther als guter antimikrobieller Wirkstoff bekannt. Siehe dazu z.B. US-Patentschriften 3 904 696, 3 629 477 und 3 800 048, CH-Patentschrift 432 119. In diesen Patentschriften sind auch verschiedene Methoden zur Herstellung dieser 2-Hydroxydiphenyläther beschrieben: a) Reduktion der Nitrogruppe eines halogenierten o-Nitrodiphenyläthers zur Aminogruppe, Diazotierung der letzteren und Substitution der Diazoniumgruppe durch die Hydroxylgruppe. Der als Ausgangsprodukt benötigte halogenierte o-Nitrodiphenyläther kann durch Kondensation eines entsprechenden 1-Nitro-2-halogenbenzols mit einem gegebenenfalls halogensubstituierten Phenol erhalten werden. b) Kondensation eines 1-Nitro-2-(bzw. 4-)halogenbenzols mit gegebenenfalls halogensubstituiertem 1-Hydroxy-2-alkoxybenzol zu einem o-(bzw. p-)Nitro-o-alkoxydiphenyläther, Entalkylierung der Alkoxygruppe, Reduktion der Nitro- zur Aminogruppe, Diazotierung der letzteren und Substitution der Diazoniumgruppe durch ein Halogenatom nach Sandmeyer, wobei die Entalkylierung auch als letzte Stufe durchgeführt werden kann. c) Kondensation eines gegebenenfalls weitere Halogenatome enthaltenden 1-Alkoxy-2-chlor(bzw. brom)-benzols mit einem gegebenenfalls halogenierten Phenolat in Gegenwart von Kupfer oder Kupfer(I)Salzen und Entalkylierung des erhaltenen o-Alkoxydiphenyläthers. d) Halogenierung von o-Hydroxydiphenyläthern, wobei ein Halogenatom in einen gegebenenfalls bereits halogenierten o-Hydroxydiphenyläther eingeführt wird.

Die vorstehend beschriebenen Verfahren a) und b) sind relativ aufwendig, da sie über viele Stufen führen und technisch aufwendige Stufen beinhalten (Hydrierung, Diazotierung). Ausserdem erhält man stark verunreinigte Produktegemische, deren Trennung und Reinigung aufwendige Operationen erfordert. Verfahren c) wird in der Schmelze durchgeführt und liefert nur bescheidene Ausbeuten. Nach Methode d) können praktisch nur bereits Halogenatome enthaltende o-Hydroxydiphenyläther halogeniert werden, da sonst meist Gemische von Produkten entstehen.

Konkret ist in der US-Patentschrift 3 904 696 die Chlorierung einiger mindestens zwei Halogenatome enthaltender 2-Phenoxyphenole zu den entsprechenden in 5-Stellung des Phenolringes substituierten Verbindungen mit Hilfe von Sulfurylchlorid in Chlorbenzol sowie die Chlorierung von 2-(4-Chlorphenoxy)-phenol zu 3,5-Dichlor-2-(4-chlorphenoxy)-phenol mit Hilfe von Chlor in Eisessig sowie die analoge Chlorierung zu einigen weiteren in 3- und 5-Stellung chlorsubstituierten 2-Phenoxyphenolen beschrieben.

In der belgischen Patentschrift 659 636 schliesslich ist die Herstellung von 5-Chlor-2-phenoxyphenol durch Chlorierung von 2-Phenoxyphenol mit Sulfurylchlorid und von 5-Chlor-2-(4-chlorphenoxy)-phenol durch Chlorierung von 2-Phenoxyanisol mit Chlor in Eisessig und anschliessender Entmethylierung beschrieben.

Es wurde nun überraschenderweise gefunden, dass man 4,5-Dichlor-2-(4-chlorphenoxy)-phenol durch selektive Chlorierung von 2-Phenoxyanisol in bestimmten Lösungsmitteln und Entmethylierung des erhaltenen 4,5-Dichlor-2-(4-chlorphenoxy)-anisols herstellen kann. Dieses neue Verfahren vermeidet die oben beschriebenen Nachteile der bisher bekannten Methoden zur Herstellung dieser Verbindung. Ausserdem fällt sie rein weiss an und weist keinen unangenehmen Fremdgeruch auf, wie dies bei Herstellung aus dem Amin via Diazotierung der Fall ist. Es ist besonders überraschend, dass die Chlorierung in einem Schritt selektiv und mit hoher Ausbeute den in 4,5,4'-Stellung trichlorsubstituierten o-Methoxydiphenyläther liefert.

Das erfindungsgemässe Verfahren zur Herstellung von 4,5-Dichlor-2-(4-chlorphenoxy)-phenol der Formel

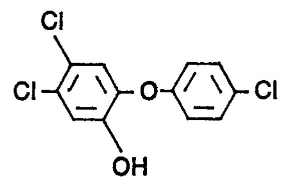

ist dadurch gekennzeichnet, dass man ein Moläquivalent 2-Phenoxyanisol bei einer Temperatur zwischen −10 und 50°C in einem niederen, mindestens ein Wasserstoffatom enthaltenden halogenierten aliphatischen Kohlenwasserstoff, einem niederen aliphatischen Nitril, in Dimethylformamid, Dimethylmethanphosphonat oder Methanol mit etwa 3-5 Moläquivalent Chlor selektiv chloriert und das erhaltene 4,5-Dichlor-2-(4-chlorphenoxy)-anisol sauer entmethyliert.

Die Erfindung betrifft auch die Herstellung des als Zwischenprodukt auftretenden 4,5-Dichlor-2-(4-chlorphenoxy)-anisols nach der ersten Stufe des oben angegebenen Verfahrens.

Niedere halogenierte, mindestens ein Wasserstoffatom enthaltende aliphatische Kohlenwasserstoffe sind vorzugsweise chlorierte Alkane oder Alkene mit 1 bis 6, insbesondere 1 bis 4 Kohlenstoffatomen. Besonders zu erwähnen sind davon Chloroform, Methylenchlorid, 1,2-Dichloräthan und Tetrachloräthan.

Niedere aliphatische Nitrile weisen vorzugsweise 2 bis 5 C-Atome auf, wovon Acetonitril und Propionitril hervorzuheben sind.

Mit Vorteil wird die Chlorierung in einem chlorierten aliphatischen Kohlenwasserstoff mit 1 bis 4 C-Atomen, einem aliphatischen Nitril mit 2 bis 5 C-Atomen, in Dimethylformamid, Dimethylmethanphosphonat oder Methanol durchgeführt.

Bevorzugte Lösungsmittel sind Dimethylformamid, Dimethylmethanphosphonat, Methanol, Acetonitril, Propionitril, Chloroform, Methylenchlorid, 1,2-Dichloräthan und Tetrachloräthan, insbesondere Dimethylformamid, Acetonitril, Methylenchlorid und 1-2-Dichloräthan.

Besonders vorteilhaft arbeitet man in halogenierten Alkanen wie Chloroform, Tetrachloräthan, vor allem aber Methylenchlorid und 1,2-Dichloräthan.

Die Temperatur während der Chlorierung kann, je nach verwendetem Lösungsmittel, zwischen −10 und 50°C liegen. Bei höheren Temperaturen bilden sich in zunehmendem Masse höher chlorierte Produkte. Im allgemeinen hält man Temperaturen zwischen 0 und 30°C ein, bevorzugt arbeitet man bei Raumtemperatur.

Bei der Chlorierung werden pro Moläquivalent 2-Phenoxyanisol etwa 3 bis 5 Moläquivalent Chlor verwendet. Grössere Mengen Chlor fördern die Bildung von höher chlorierten Nebenprodukten, geringere Mengen verringern die Ausbeute. Bevorzugt wird eine Menge von etwa 4 Moläquivalent Chlor pro Moläquivalent 2-Phenoxyanisol eingesetzt.

Man kann dem Reaktionsgemisch vor der Chlorierung noch katalytische Mengen an verschiedenen Stoffen beigeben, wobei in manchen Fällen eine geringfügige Erhöhung der Ausbeute zu beobachten ist, Derartige Stoffe sind zum Beispiel Jod, Triäthylamin und verschiedene Salze wie z.B. $FeCl_3$, $NiCl_2$ und $CoCl_2$.

Die Entmethylierung (Ätherspaltung) des 4,5--Dichlor-2-(4-chlorphenoxy)-anisols kann nach an sich bekannten Methoden erfolgen. Als Säuren kommen Protonensäuren, wie z.B. Halogenwasserstoffsäuren, insbesondere Bromwasserstoffsäure, oder Lewis-Säuren, wie z.B. $BF_3$, und insbesondere $AlCl_3$ in Frage. Die Reaktion wird in einem inerten Lösungsmittel durchgeführt. Bei Verwendung von Halogenwasserstoffsäuren arbeitet man vorzugsweise in wässrigem Medium, bei Verwendung von Lewis-Säuren in organischen Lösungsmitteln. Bevorzugt wird mit Hilfe von $AlCl_3$ in Benzol als Lösungsmittel entmethyliert [siehe US-Patentschrift 3 629 477 (Beispiel 40) und CH-Patentschrift 428 759].

Die beiden Stufen des erfindungsgemässen Verfahrens (Chlorierung und Ätherspaltung) können auch ohne Isolierung des Zwischenproduktes in Form eines Eintopfverfahrens ausgeführt werden, wobei gegebenenfalls das bei der Chlorierung verwendete Lösungsmittel abgedampft und durch ein anderes ersetzt wird. Vorzugsweise jedoch wird das Zwischenprodukt isoliert.

Da die Chlorierung ausserordentlich selektiv verläuft, kann das gewünschte 4,5-Dichlor-2-(4--chlorphenoxy)-anisol leicht aus dem Reaktionsgemisch isoliert werden, ohne dass langwierige Trennungen von Isomeren und/oder anderen Chlorierungsprodukten nötig sind. Meist fällt das Produkt direkt aus dem verwendeten Lösungsmittel aus.

Die Herstellung des als Ausgangsprodukt benötigten 2-Phenoxyanisols kann zum Beispiel leicht durch Umsetzung von Guajacol (2-Methoxyphenol) mit Brombenzol nach der Methode von H.E. Ungnade und E.F. Orwoll, Organic Synthesis Vol. 3, 566 (1955) erfolgen.

Das nach dem erfindungsgemässen Verfahren herstellbare 4,5-Dichlor-2-(4-chlorphenoxy)--phenol zeichnet sich, wie bereits eingangs erwähnt, durch gute antimikrobielle Eigenschaften aus. Es kann daher zu Desinfektionszwekken sowie zum Schützen verschiedenster Substrate vor dem Befall durch Bakterien und Pilze verwendet werden. Siehe dazu US-Patentschriften 3 800 048, 3 629 477 und 3 904 696, CH-Patentschrift 432 119.

In den nachfolgenden die Erfindung erläuternden Beispielen bedeuten Prozentangaben Gewichtsprozent, soweit nicht anders angegeben.

Beispiel 1

In eine Lösung von 84,2 g (0,42 Mol) 2-Phenoxyanisol in 500 ml Dimethylformamid leitet man unter Rühren während 70 Minuten 115 g (1,62 Mol) Chlor bei einer Temperatur von 20 bis 25°C ein. Während des Einleitens wird das Reaktionsgefäss mit Eis gekühlt. Dann giesst man die Reaktionslösung unter Rühren auf 3 Liter Eiswasser, worauf nach etwa 5 Minuten das Produkt auskristallisiert. Nach weiteren 45 Minuten werden die Kristalle abfiltriert, mit 1 Liter Wasser gewaschen und aus 2,2 Liter Methanol umkristallisiert. Das so erhaltene 4,5-Dichlor-2-(4--chlorphenoxy)-anisol weist einen Schmelzpunkt von 105 bis 106°C auf.

Wird der Lösung vor Beginn der Chloreinleitung eine katalytische Menge (z.B. 0,1 bis 5 g) an $FeCl_3$, $NiCl_2$ oder $CoCl_2$ zugegeben und verfährt man sonst wie beschrieben, erhält man ähnliche Resultate.

Beispiel 2

In eine Lösung von 8 g (0,04 Mol) 2-Phenoxyanisol in 75 ml Acetonitril werden bei Raumtemtemperatur während 20 Minuten unter Rühren 11,5 g (0,16 Mol) Chlor eingeleitet; anschliessend wird das Reaktionsgemisch noch 70 Minuten bei Raumtemperatur weitergerührt. Die weisse Suspension wird auf 0°C abgekühlt, die gebildeten weissen Kristalle [4,5-Dichlor-2-(4--chlorphenoxy)-anisol] werden abfiltriert und zweimal mit je 10 ml eiskaltem Acetontril gewaschen. Aus dem Filtrat kann weiteres 4,5-Dichlor-2-(4-chlorphenoxy)-anisol isoliert werden. Nach Umkristallisation aus Methanol schmilzt das Produkt bei 105°C.

Beispiel 3

In eine Lösung von 8 g (0,04 Mol) 2-Phenoxyanisol in 75 ml Acetonitril werden bei Raumperatur unter Rühren während $1^{1}/_{2}$ Stunden 13 g (0,183 Mol) Chlor eingeleitet; anschliessend wird die Mischung bei Raumtemperatur noch 30 Minuten gerührt. Nach Abkühlen auf 0°C werden die gebildeten Kristalle abfiltriert und aus Methanol umkristallisiert, worauf man 4,5-Dichlor-2-(4-chlorphenoxy)-anisol in Form von weissen Kristallen mit einem Schmelzpunkt von 105°C erhält.

Beispiel 4

In eine Lösung von 8 g 2-Phenoxyanisol in 85 ml Chloroform werden bei Raumtemperatur unter Rühren während 25 Minuten 11,2 g Chlor eingeleitet; danach wird die Mischung bei Raumtemperatur noch 30 Minuten gerührt. Nach Abdampfen des Lösungsmittels und Umkristallisieren des kristallinen Rückstandes aus Methanol erhält man 4,5-Dichlor-2-(4-chlorphenoxy)-anisol in Form von weissen Kristallen mit einem Schmelzpunkt von 105 bis 106°C.

Beispiel 5

In eine Lösung von 8 g 2-Phenoxyanisol in 85 ml Methylenchlorid werden bei Raumtemperatur unter Rühren innerhalb von 20 Minuten 11,2 g Chlor eingeleitet; anschliessend wird das Reaktionsgemisch eine weitere Stunde bei Raumtemperatur gerührt. Nach Abdampfen des Lösungsmittels und Umkristallisieren des kristallinen Rückstandes aus Methanol erhält man 4,5-Dichlor-2-(4-chlorphenoxy)-anisol in Form von weissen Kristallen mit einem Schmelzpunkt von 105 bis 106°C.

Werden der Lösung vor Beginn der Chloreinleitung 0,5 g Triäthylamin oder 50 mg Jod zugegeben, so erhält man ähnliche Resultate.

Beispiel 6

In eine Lösung von 8 g 2-Phenoxyanisol in 85 ml 1,2-Dichloräthan werden bei Raumtemperatur unter Rühren innerhalb von 20 Minuten 11,5 g Chlor eingeleitet; anschliessend wird das Reaktionsgemisch noch 30 Minuten bei Raumtemperatur gerührt. Nach Abdampfen des Lösungsmittels und Umkristallisieren des kristallinen Rückstandes aus Methanol erhält man 4,5-Dichlor-2-(4-chlorphenoxy)-anisol in Form von weissen Kristallen mit einem Schmelzpunkt von 105 bis 106°C.

Beispiel 7

In eine Lösung von 8 g 2-Phenoxyanisol und 0,3 g Eisen(III)chlorid in 85 ml Dimethylformamid werden unter Rühren bei Raumtemperatur innerhalb 35 Minuten 11,5 g Chlor eingeleitet; anschliessend wird das Reaktionsgemisch noch 30 Minuten lang gerührt. Nach Verdünnen mit 200 ml Wasser werden die ausgefallenen Kristalle abfiltriert und aus Methanol umkristallisiert. Man erhält so 4,5-Dichlor-2-(4-chlorphenoxy)-anisol in Form weisser Kristalle mit einem Schmelzpunkt von 105°C.

Beispiel 8

In eine Lösung von 8 g 2-Phenoxyanisol in 50 ml Dimethylmethanphosphonat werden unter Rühren bei einer Temperatur von 25 bis 30°C innerhalb von 30 Minuten 11,5 g Chlor eingeleitet; anschliessend wird das Reaktionsgemisch noch $1^{1}/_{2}$ Stunden gerührt. Das Lösungsmittel wird im Vakuum (12 Torr) bei 90°C entfernt und der Rückstand aus Methanol umkristallisiert. Man erhält so 4,5-Dichlor-2-(4-chlorphenoxy)-anisol in Form weisser Kristalle mit einem Schmelzpunkt von 105 bis 106°C.

Beispiel 9

Zu einer Lösung von 124 g (0,4 Mol) 4,5-Dichlor-2-(4-chlorphenoxy)-anisol (erhalten nach einem der Beispiele 1 bis 8) in 520 ml Benzol gibt man 157,4 g (1,1 Mol) Aluminiumchlorid und erhitzt die Mischung unter Rühren 45 Minuten lang zum Rückfluss. Dann giesst man die auf Raumtemperatur abgekühlte Suspension unter Rühren auf eine Mischung von 970 g Eis und 970 ml konzentrierte Salzsäure. Die Phasen werden im Scheidetrichter getrennt und die Benzolphase viermal mit je 250 ml 5%iger Kochsalzlösung gewaschen. Dann giesst man die Benzolphase zu 1 Liter 2 N wässriger Natriumhydroxidlösung und 3 l Wasser und erwärmt die Suspension unter Rühren auf 60°C. Dann trennt man die Benzolphase ab und vertreibt das restliche Benzol aus der wässrigen Phase durch Einleiten von Wasserdampf. Die wässrige Lösung wird mit Tierkohle versetzt und filtriert. Zum Filtrat gibt man 400 ml konzentrierte Salzsäure, filtriert die ausgefallenen Kristalle ab und wäscht sie mit 1,5 l Wasser. Man erhält so 111,6 g 4,5-Dichlor-2-(4-chlorphenoxy)-phenol in Form weisser Kristalle mit einem Schmelzpunkt von 92 bis 93,5°C.

**Patentansprüche**

1. Verfahren zur Herstellung von 4,5-Dichlor-2-(4-chlorphenoxy)-phenol, dadurch gekennzeichnet, dass man 1 Moläquivalent 2-Phenoxyanisol bei einer Temperatur zwischen −10 und 50°C in einem niederen, mindestens ein Wasserstoffatom enthaltenden halogenierten aliphatischen Kohlenwasserstoff, einem niederen aliphatischen Nitril, in Dimethylformamid, Dimethylmethanphosphonat oder Methanol mit 3 bis 5 Moläquivalent Chlor selektiv chloriert und das erhaltene 4,5-Dichlor-2-(4-chlorphenoxy)-anisol sauer entmethyliert.

2. Verfahren zur Herstellung von 4,5-Dichlor-2-(4-chlorphenoxy)-anisol, dadurch gekennzeichnet, dass man 1 Moläquivalent 2-Phenoxyanisol bei einer Temperatur zwischen −10 und 50°C in einem niederen, mindestens ein Wasserstoffatom enthaltenden halogenierten aliphatischen Kohlenwasserstoff, einem niederen aliphatischen Nitril, in Dimethylformamid, Dimethylmethanphosphonat oder Methanol selektiv mit 3 bis 5 Moläquivalent Chlor chloriert.

3. Verfahren nach Anspruch 1 oder 2, dadurch gekennzeichnet, dass man die Chlorierung in einem chlorierten, mindestens ein Wasserstoffatom enthaltenden aliphatischen Kohlenwasserstoff mit 1 bis 4 C-Atomen, einem aliphatischen Nitril mit 2 bis 5 C-Atomen, in Dimethylformamid, Dimethylmethanphosphonat oder Methanol durchführt.

4. Verfahren nach Anspruch 3, dadurch gekennzeichnet, dass man als Lösungsmittel Dimethylformamid, Dimethylmethanphosphonat, Methanol, Acetonitril, Propionitril, Chloroform, Methylenchlorid, 1,2-Dichloräthan oder Tetrachloräthan verwendet.

5. Verfahren nach Anspruch 4, dadurch gekennzeichnet, dass man als Lösungsmittel Dimethylformamid, Acetonitril, Methylenchlorid oder 1,2-Dichloräthan verwendet.

6. Verfahren nach einem der Ansprüche 1 bis 5, dadurch gekennzeichnet, dass man die Chlorierung bei einer Temperatur zwischen 0 und 30°C durchführt.

7. Verfahren nach Anspruch 1, dadurch gekennzeichnet, dass man die Entmethylierung in einem inerten Lösungsmittel mit Hilfe einer Protonensäure, vorzugsweise einer Halogenwasserstoffsäure oder einer Lewis-Säure, insbesondere $AlCl_3$ oder $BF_3$ durchführt.

8. Verfahren nach Anspruch 7, dadurch gekennzeichnet, dass mal als Säure $AlCl_3$ verwendet.

**Claims**

1. A process for the manufacture of 4,5-dichloro-2-(4-chlorophenoxy)-phenol, which comprises selectively chlorinating one molar equivalent of 2-phenoxyanisole at a temperature between −10° and +50°C, in a lower halogenated aliphatic hydrocarbon which contains at least one hydrogen atom, a lower aliphatic nitrile, in dimethyl formamide, dimethylmethanephosphonate or methanol, with 3 to 5 molar equivalents of chlor ne, and demethylating the 4,5-dichloro-2-(4-chlorophenoxy)-anisole obtained with an acid.

2. A process for the manufacture of 4,5-dichloro-2-(4-chlorophenoxy)-anisole, wherein 1 molar equivalent of 2-phenoxyanisole is selectively chlorinated with 3 to 5 molar equivalents of chlorine at a temperature between −10° and 50°C in a lower halogenated aliphatic hydrocarbon which contains at least one hydrogen atom, a lower aliphatic nitrile, in dimethyl formamide, dimethylmethanephosphonate or methanol.

3. A process according to either of claims 1 or 2, wherein the clorination is carried out in a chlorinated aliphatic hydrocarbon containing 1 to 4 carbon atoms and at least one hydrogen atom, an aliphatic nitrile containing 2 to 5 carbon atoms, in dimethyl formamide, dimethylmethanephosphonate or methanol.

4. A process according to claim 3, wherein the solvent employed is dimethyl formamide, dimethylmethanephosphonate, methanol, acetonitrile, propionitrile, chloroform, methylene chloride, 1,2-dichloroethane or tetrachloroethane.

5. A process according to claim 4, wherein the solvent employed is dimethyl formamide, acetonitrile, methylene chloride or 1,2-dichloroethane.

6. A process according to any one of claims 1 to 5, wherein the chlorination is carried out in the temperature range between 0° and 30°C.

7. A process according to claim 1, wherein the demethylation is carried out in an inert solvent with a proton acid, preferably a hydrohalic or a Lewis acid, especially $AlCl_3$ or $BF_3$.

8. A process according to claim 7, wherein the acid employed is $AlCl_3$.

**Revendications**

1. Procédé pour la préparation de 4,5-dichloro-2-(4-chlorophénoxy)-phénol, caractérisé par le fait qu'on chlore sélectivement un équivalent molaire de 2-phénoxyanisol, à une température comprise entre −10° et 50°C, avec 3 à 5 équivalents molaires de chlore, dans un hydrocarbure aliphatique inférieur, halogéné, contenant au moins un atome d'hydrogène, dans un nitrile aliphatique inférieur, dans le diméthylformamide, dans le diméthylméthanephosphonate ou dans le méthanol, et que le 4,5-dichloro-2-(4-chlorophénoxy)-anisol obtenu est déméthylé d'une façon acide.

2. Procédé pour la préparation du 4,5-dichloro-2-(4-chlorophénoxy)-anisol, caractérisé par le fait qu'on chlore un équivalent molaire de 2-phénoxyanisol, à une température comprise entre −10° et 50°C, sélectivement avec 3 à 5 équivalents molaires de chlore, dans un hydrocarbure aliphatique inférieur, halogéné, contenant au moins un atome d'hydrogène, dans un nitrile aliphatique inférieur, dans le diméthylformamide, dans le diméthylméthanephosphonate ou dans le méthanol.

3. Procédé selon les revendications 1 ou 2, caractérisé par le fait qu'on effectue la chloration dans un hydrocarbure aliphatique, chloré, contenant au moins un atome d'hydrogène, ayant 1 à 4 atomes de carbone, dans un nitrile aliphatique ayant 2 à 5 atomes de carbone, dans le diméthylformamide, dans le diméthylméthanephosphonate ou dans le méthanol.

4. Procédé selon la revendication 3, caractérisé par le fait qu'on utilise comme solvant: le diméthylformamide, le diméthylmethanephosphonate, le méthanol, l'acétonitrile, le propionitrile, le chloroforme, le chlorure de méthylène, le 1,2-dichloréthane ou le tétrachloréthane.

5. Procédé selon la revendication 4, caractérisé par le fait qu'on utilise comme solvant: le diméthylformamide, l'acétonitrile, le clorure de méthylène ou le 1,2-dichloréthane.

6. Procédé selon l'une quelconque des revendications 1 à 5, caractérisé par le fait qu'on effectue la chloration à une température comprise entre 0° et 30°C.

7. Procédé selon la revendication 1, caractérisé par le fait qu'on effectue la déméthylation dans un solvant inerte à l'aide d'un acide protonique, de préférence un hydracide halogéné ou un acide de Lewis, en particulier $AlCl_3$ ou $BF_3$.

8. Procédé selon la revendication 7, caractérisé par le fait qu'on utilise comme acide: $AlCl_3$.